# EUROPEAN PATENT SPECIFICATION

(11) **EP 1 336 401 B1**
(45) Date of publication and mention of the grant of the patent: **08.08.2007**
(21) Application number: 02293039.0
(22) Date of filing: 09.12.2002
(51) Int. Cl.: A61K 8/81, A61K 8/86, A61Q 5/06, C08L 33/00

(54) **Reshapable hair styling non rinsed compositions comprising (meth)acrylic copolymers**
Leave-on, umformbare Haarformungszusammensetzungen enthaltend (Meth)acrylat copolymere
Compositions de coiffage repositionnables non-rincées comprenant des copolymères (méth)acryliques

(30) Priority: 20.12.2001 US 22253
(43) Date of publication of application: 20.08.2003
(73) Proprietor: L'ORÉAL, 75008 Paris (FR)
(72) Inventor: Rollat-Corvol, Isabelle, 75017 Paris (FR); Samain, Henri, 91570 Bievres (FR)
(74) Representative: Bourdeau, Françoise

(56) References cited:
- EP-A- 0 761 199
- EP-A- 0 985 401
- EP-A- 1 174 113
- EP-A- 1 288 258
- WO-A-00/57846
- WO-A-02/09656
- WO-A-99/08652
- FR-A- 2 760 360
- US-A- 4 196 190
- US-A- 5 501 851
- US-A- 5 658 558
- US-A- 6 132 705
- US-A- 6 149 898
- US-B1- 6 214 328
- US-B1- 6 294 158

## Description

The present invention relates to a reshapable hair styling composition. In particular, the present invention relates to a reshapable hair styling composition that is a non-rinse composition.

Fixing the hairstyle is an important element in hair styling, and involves maintaining a shaping that has already been carried out, or simultaneously shaping and fixing the hair. In accordance with the invention, the term "hair styling composition" relates to any kind of hair composition that can be used to effect hair styling.

The most prevalent hair styling compositions on the cosmetic market for shaping and/or maintaining the hairstyle are spray compositions comprising a solution, usually alcohol- or water-based, and one or more materials, generally polymer resins. One of the functions of polymer resins is to form links between the hairs. These materials, also called fixatives, are often found in a mixture with various cosmetic adjuvants. This solution is generally packaged either in an appropriate aerosol container, which is pressurized with the aid of a propellant, or in a pump flask.

Some other known hair styling compositions include styling gels and mousses, which are generally applied to the wetted hair before brushing or setting it. In contrast to conventional aerosol lacquers, certain of these other known hair styling compositions disadvantageously are not designed to allow the hair to be fixed in a shape created before their application. In fact, these compositions are essentially aqueous and their application wets the hair; hence these compositions are not designed to maintain the initial shape of the hairstyle. In order to shape and fix the hairstyle, therefore, it is necessary to carry out subsequent brushing and/or drying with these types of compositions.

Thus, many hair styling compositions exist that have the same disadvantage: they are not designed to allow the hairstyle to be later modified to a desired shape, which is other than that formed initially, without starting the styling and fixing operations again. Moreover, under various kinds of stress, the hairstyle has a tendency to take on an undesirable permanent set, which cannot easily be modified. Also in the styling process, one may desire hair conditioning benefits, such as ease of combing and soft hair feel appearance.
WO 02/09656, objectionable under Art. 54(3) EPC, discloses a reshapable hair styling composition comprising at least one (meth)acrylic copolymer, wherein said at least one (meth)acrylic copolymer comprises (see Claim 1 and ex. 1-2):
(a) units derived from at least one monomer chosen from butyl (meth)acrylate monomers,
(b) units derived from at least one monomer chosen from hydroxy alkyl (meth)acrylate monomers, and
wherein said composition provides a reshapable effect and is a non-rinse composition (page 13, line 18).

A subject of the invention is a reshapable hair styling composition comprising, optionally in a cosmetically acceptable vehicle, at least one (meth)acrylic copolymer, wherein the at least one (meth)acrylic copolymer comprises: (a) units derived from n-butyl acrylate monomer, (b) units derived from 2-hydroxy ethyl methacrylate monomer, and (c) units derived from 2-ethyl hexylacrylate monomer, wherein said composition provides a reshapable effect and is a non-rinse composition.

Preferred is when the at least one (meth)acrylic copolymer comprises: (a) from about 10 to about 90 weight percent of units derived from at least n-butyl acrylate monomer, (b) from about 2 to about 50 weight percent of units derived from 2-hydroxy ethyl methacrylate and (c) units derived from at least 2-ethyl hexyl acrylate monomer, wherein said composition provides a reshapable effect and is a non-rinse composition.

Particularly preferred is when (a) is from about 10 to about 90 weight percent , (b) is from about 2 to about 50 weight percent and (c) is up to about 80 weight percent.

Still more preferred are the values (a) from about 30 to about 40 weight percent (b) from about 2 to about 10 weight percent and (c) from about 50 to about 70 weight percent.

The weight percentages of the (a), (b), and (c) units is based on the total weight of each monomer type used compared to the total weight of all monomers used.

Another subject of the invention is a reshapable hair styling composition comprising at least one (meth)acrylic copolymer, as described above, wherein said reshapable hair styling composition is a non-rinse composition in the form of a spray, aerosol, mousse, gel, lotion, cream, dispersion, or emulsion.

Another subject of the invention is an aerosol device comprising a vessel, which comprises: (1) an aerosol composition, which provides a reshapable effect and comprises a liquid phase comprising at least one composition comprising at least one (meth)acrylic copolymer, as described above, wherein said composition is a non-rinse composition, and a propellant, and (2) a dispenser.

Another subject of the invention is a method of cosmetically treating hair, comprising applying to the hair before, during, or after shaping of a hairstyle of said hair a composition comprising at least one (meth)acrylic copolymer, as described above, wherein said composition provides a reshapable effect and is a non-rinse composition.

The term "(meth)acrylate" is used to encompass both the terms acrylate and methacrylate. Similarly, the term "(meth)acrylic acid" is used to encompass both acrylic acid and methacrylic acid. As used herein, the term "polyfunctional crosslinking agent" is used to mean a crosslinking agent having an average functionality greater than 1, such as greater than 1.8, and further such as about 2.0 or greater. But the average functionality is less than about 6, such as less than about 4, and further such as about 3 or less. The term "non-rinse composition" is used to mean any composition that is formulated so as not to be rinsed off after application to the hair.

The non-rinse composition may be in any of the conventional forms of non-rinse cosmetic compositions including, but not limited to, permanent waving compositions, waving compositions, hair dye compositions, hair fixing products, hair styling gel products, products to use before or after a hair dye treatment, products to use before or after a permanent waving treatment, hair straightening compositions, products to use before or after a hair straightening treatment, fixing foams, and combinations thereof.

The term "reshapable" hair styling composition means a hair styling composition providing hair styling that can be restored or modified without new material or heat being applied. For example, in order to restore or modify the hairstyle in case of "drooping" or loss of setting (dishevelment), no new materials, such as water or any form of fixing agent, or heat are required. Thus, to provide a "reshapable" effect means to provide a hair styling that can be restored or modified without new material or heat being applied. The efficacy of the composition can be long lasting, such as 10-24 hours, giving rise to a durable styling effect. Other terms, which may be synonymous with reshapable, include repositionable, remoldable, restyleable, rearrangable, and remodelable.

In one embodiment of the invention, the (meth)acrylic copolymer of such reshapable hair styling compositions may be in the form of an emulsion or dispersion. All emulsions comprise a continuous phase and at least one dispersed phase. The term "dispersion" means generally a multi-phase system where at least one phase contains discrete particles distributed throughout a bulk substance. A portion of the polymer may exist as the discrete particle in an aqueous phase. Dispersions are possible through the use of certain components that are insoluble in the aqueous system. By "dispersion," it is also meant that not necessarily the entire polymer needs to be water insoluble; some of the polymer can be soluble in the aqueous mixture. It may be desirable that a dispersion remains stable under ambient conditions. In one embodiment, dispersions are stable at room temperature for more than about 30 days, such as for more than about 90 days, for more than about 180 days, and for more than about 360 days. A dispersion is deemed stable so long as the discrete particles of the internal phase remain distributed throughout the bulk substance (external phase).

In one embodiment, such dispersions may be blended with other dispersions or with other known additives such as fillers, plasticizers, pigments (such as carbon black), silica sols and other known leveling agents, wetting agents, antifoaming agents, and stabilizers.

In general, the monomer recited in (a) constitutes, for example, from about 10 to about 90 weight percent of the total amount of monomers used. In one embodiment, it may constitute from about 15 to about 50 weight percent of the total amount of monomers used. In a separate embodiment, it may constitute from about 50 to about 90 weight percent of the total amount of monomers used, such as from about 70 to about 90 weight percent of the total amount of monomers used. The monomer recited in (a) is n-butyl (meth)acrylate.

In general, the monomers recited in (b) constitute, for example, from about 2 to about 50 weight percent of the total amount of monomers used. In one embodiment, they may constitute from about 2 to about 25 weight percent of the total amount of monomers used. In a separate embodiment, they may constitute from about 10 to about 50 weight percent of the total amount of monomers used, such as from about 10 to about 30 weight percent of the total amount of monomers used. The monomer recited in (b) is 2-hydroxy ethyl methacrylate.

In general, the monomers recited in (c) constitute for example, up to about 80 weight percent of the total amount of monomers used. In one embodiment, they may constitute from about 30 to about 80 weight percent of the total amount of monomers used. In a separate embodiment, they may constitute up to about 50 weight percent of the total amount of monomers used, such as up to about 30 weight percent of the total amount of monomers used. The at least one co-polymerizable monomer recited in (c) is 2-ethyl hexyl (meth)acrylate. In another embodiment, the units derived from 2-ethyl hexyl (meth)acrylate monomers constitute from about 30 to about 80 weight percent of the total amount of monomers used.

One or more polyfunctional crosslinking agents may be included. Examples of crosslinking agents include but are not limited to those chosen from divinylbenzene, alkyl diacrylates (such as those chosen from 1,2-ethylene glycol diacrylate, 1,4-butanediol diacrylate, 1,6-hexanediol diacrylate, 1,8-octanediol diacrylate, and 1,12-dodecanediol diacrylate), alkyl triacrylates and alky tetracrylates (such as trimethylol propane triacrylate and pentaerythritol tetraacrylate), monoethylenically unsaturated aromatic ketones (such as 4-acryloxybenzophenone), multifunctional aziridine amides (such as 1,1'-(1,3-phenylenedicarbonyl)bis[2-methyl aziridine], 2,2,4-trimethyladipoyl bis [2-ethyl aziridine], 1, 1'-azelaoyl bis [2-methyl aziridine], and 2,4,6-tris(2-ethyl-1-aziridinyl)-1,3,5 triazine), metal ion crosslinkers (such as copper, zinc, zirconium, and chromium) and mixtures thereof. In one embodiment, the metal ion crosslinkers are chosen from chelated esters of ortho-titanic acid sold under the tradename TYZOR and commercially available from the E.I. du Pont de Numours Co. In another embodiment, the TYZOR is TYZOR AA, which is titanium acetyl acetonate. In yet another embodiment, the crosslinking agent is 1,6-hexanediol diacrylate.

Crosslinking agents, when used, comprise up to about 10 parts by weight, typically about 0.1 to about 2 parts by weight of the total copolymerizable mixture based on 100 parts by weight of the monomers recited in (a), (b), and (c), when present.

In one embodiment, the copolymer may be optionally obtained with one or more water-soluble and/or oil-soluble initiators, which are useful in preparing (meth)acrylic emulsions. Such initiators, on exposure to heat, generate free-radicals which initiate (co)polymerization of the butyl (meth)acrylate monomers, hydroxyalkyl (meth)acrylate monomers, and the optional comonomer and crosslinking agent components. In one embodiment, one or more water soluble initiators are used. Suitable water-soluble initiators include but are not limited to those chosen from potassium persulfate, ammonium persulfate, sodium persulfate, and mixtures thereof; oxidation-reduction initiators such as the reaction product of the above-mentioned persulfates and reducing agents such as those chosen from sodium metabisulfite and sodium bisulfite; and 4,4'-azobis(4-cyanopentanoic acid) and its soluble salts (e.g., sodium, potassium). In another embodiment, the water-soluble initiator is potassium persulfate.

Suitable oil-soluble initiators include but are not limited to those chosen from azo compounds such as VAZO 64 (2,2'-azobis(isobutyronitrile) and VAZO 52 (2,2'-azobis(2,4-dimethylpentanenitrile)), both available from E.I. du Pont de Numours Co.; and peroxides such as benzoyl peroxide, lauroyl peroxide, and mixtures thereof. In one embodiment, the oil-soluble thermal initiator is 2,2'-azobis(isobutyronitrile). When used, initiator(s) may comprise from about 0.05 to about 1 parts by weight, also about 0.1 to about 0.5 parts by weight based on 100 parts by weight of the total copolymerizable mixture

In another embodiment, the copolymer may be optionally obtained with one or more chain transfer agents. Examples of useful chain transfer agents include but are not limited to those chosen from carbon tetrabromide, alcohols, mercaptans, and mixtures thereof. In one embodiment, the chain transfer agent is chosen from isooctylthioglycolate and carbon tetrabromide. The copolymerizable mixture may further comprise up to about 0.5 parts by weight of one or more chain transfer agents, typically about 0.01 weight percent to about 0.5 parts by weight, if used, also about 0.05 parts by weight to about 0.2 parts by weight, based upon 100 parts by weight of the total copolymerizable mixture.

Polymerization via emulsion techniques may require the presence of one or more emulsifiers (which may also be called emulsifying agents or surfactants). Useful emulsifiers for the present invention include those chosen from anionic surfactants, nonionic surfactants, and mixtures thereof.

Useful anionic surfactants, as emulsifiers, include but are not limited to those whose molecular structure includes at least one hydrophobic moiety chosen from (about C₆ to about C₁₂) alkyls, (about C₆ to about C₁₂) alkyl aryls, and (about C₆ to about C₁₂) alkenyls and at least one anionic group chosen from sulfates, sulfonates, phosphates, polyoxyethylene sulfates, polyoxyethylene sulfonates, polyoxyethylene phosphates, and the like, and the salts of such groups. In one embodiment, said salts are chosen from alkali metal salts, ammonium salts, tertiary amino salts, and the like. Representative commercial examples of useful anionic surfactants, as emulsifiers, include sodium lauryl sulfates, available from Stepan Chemical Co. as POLYSTEP B-3; sodium lauryl ether sulfates, available from Stepan Chemical Co. as POLYSTEP B-12; sodium dodecyl benzene sulfonates, available from Rhône-Poulenc as SIPONATE DS-10; and alkylene polyalkoxy ammonium sulfates, available from PPG Industries as MAZON SAM-211.

Useful nonionic surfactants, as emulsifiers, include but are not limited to those whose molecular structure comprises a condensation product of an organic aliphatic and/or alkyl aromatic hydrophobic moiety with a hydrophilic alkylene oxide such as ethylene oxide. The HLB (Hydrophilic-Lipophilic Balance) of useful nonionic surfactants, as emulsifiers, is about 10 or greater, such as from about 10 to about 20. The HLB of a surfactant is an expression of the balance of the size and strength of the hydrophilic (water-loving or polar) groups and the lipophilic (oil-loving or non-polar) groups of the surfactant. Commercial examples of nonionic surfactants useful in the present invention include but are not limited to nonylphenoxy or octylphenoxy poly(ethyleneoxy) ethanols available from Rhône-Poulenc as the IGEPAL CA or CO series, respectively; C₁₁-C₁₅ secondary-alcohol ethoxylates available from Union Carbide as the TERGITOL 15-S series; and polyoxyethylene sorbitan fatty acid esters available from ICI Chemicals as the TWEEN series of surfactants.

In one embodiment, an emulsion polymerization of this invention is carried out in the presence of one ore more anionic surfactants, as emulsifiers. A useful range of emulsifier concentration is from about 0.5 to about 8 weight percent, such as from about 1 to about 5 weight percent, based on the total weight of all monomers.

In one embodiment, the (meth)acrylic copolymers are (meth)acrylic emulsions or dispersions. (Meth)acrylic emulsions and dispersions may be prepared by a semi-continuous emulsion polymerization process. In the process, a flask is charged with a seed monomer mixture comprising deionized (Dl) water, surfactant, butyl (meth)acrylate monomers recited in (a), hydroxyalkyl (meth)acrylate monomers recited in (b), and the optional components such as co-polymerizable monomers recited in (c), polyfunctional crosslinking agents, chain transfer agents, pH modifiers, and other additives. The mixture is stirred and heated under an inert atmosphere such as a nitrogen blanket. When the mixture has reached induction temperature, typically about 50 °C to about 70 °C, the first initiator is added to initiate the polymerization and the reaction is allowed to exotherm. After the seed reaction is completed, the batch temperature is then raised to the feed reaction temperature, about 70 °C to about 85 °C. At the feed reaction temperature, the monomer pre-emulsion comprising Dl water, surfactant, butyl (meth)acrylate monomers recited in (a), hydroxyalkyl (meth)acrylate monomers recited in (b), and the optional components such as co-polymerizable monomers recited in (c), polyfunctional crosslinking agents, chain transfer agents, and other additives is added to the stirred flask over a period of time, typically 2 to 4 hours, while the temperature is maintained. At the end of the feed reaction, the second initiator charge, if used, is added to the reaction to further reduce residual monomers in the emulsion/dispersion. After an additional hour of heating, the mixture is cooled to room temperature (about 23 °C) and the emulsion/dispersion is collected for evaluation.

In one embodiment, the pH of the emulsion/dispersion prepared using this method is about 2 to about 3. The acidity of the emulsion/dispersion can be modified following emulsion/dispersion formation using one or more pH modifiers such as a basic solutions (e.g., solutions of sodium hydroxide, ammonium hydroxide and the like) or buffer solutions (e.g., sodium bicarbonate and the like), to less acidic levels. In another embodiment, the pH is 7 or less. In yet another embodiment, the pH is in the range of 2 to 6.

In one embodiment of the invention, the (meth)acrylic copolymers may be neutralized in the emulsion/dispersion and/or in the composition by one or more neutralizing agents. Suitable neutralizing agents may be chosen from organic, inorganic, and organomineral bases, such as amino methyl propanols, sodium and potassium hydoxides, primary, secondary and tertiary amines, ammoniacs, derivatives thereof, and combinations thereof.

In one embodiment, the (meth)acrylic emulsions of the invention may also contain one or more conventional additives, such as plasticizers, dyes, fillers, antioxidants, and UV stabilizers. Such additives can be used if they do not adversely affect the reshapable properties of the composition.

In yet another embodiment of the invention, the (meth)acrylic copolymer has a glass transition temperature (Tg) ranging from about -100 °C to about 15 °C. According to the present invention, the Tg of the (meth)acrylic copolymer is obtained following the application of the (meth)acrylic copolymer in a aqueous or hydroalcoholic medium to a matrix and then drying until the weight does not change. The glass transition temperature is determined by the Differential Scanning Calorimetric method (DSC).

In one embodiment of the invention, the (meth)acrylic copolymer is chosen from nonionic and weakly anionic (meth)acrylic copolymers. Weakly anionic means that the level of units derived from anionic monomers in the copolymer is less than about 5% by weight.

In one embodiment of the invention, the (meth)acrylic copolymer may be present in an amount ranging from about 0.01 to about 40, such as from about 0.1 to about 15, weight percent of the total weight of the composition in order to provide a reshapable effect.

The composition may further comprise any cosmetically acceptable vehicle that does not substantially interfere with the adhesive properties of the at least one (meth)acrylic copolymer. The choice of vehicle is adapted to the method of application selected. In one embodiment, the cosmetically acceptable vehicle may be chosen from water, water miscible solvents such as lower alcohols, e.g., C₁ to C₄ branched and straight chain aliphatic alcohols, and combinations thereof. In one embodiment, the at least one adhesive particle is insoluble in the cosmetically acceptable vehicle.

The vehicle may also comprise one or more additional solvents. For example, other rapid evaporating solvents may be used, such as hexamethyldisiloxane (HMDS); cyclic silicones (D₄ and D₅); C₄-C₁₀ alkanes including isoparafins such as Permethyl 97A and Isopar C; acetone; hydrofluoroethers (HFEs) and the like.

The composition according to the invention may further comprise at least one constituent known in the cosmetic arts that does not substantially interfere with the reshapable properties of the at least one (meth)acrylic copolymer. Such constituents may be chosen from, but are not limited to: reducing agents (such as thiols); silanes (such as aminopropyl triethoxy silane); fatty substances; thickeners; plasticizers; anti-foaming agents; hydrating agents; fillers; sunscreens (such as UV filters); active haircare agents; perfumes; preservatives; cationic, anionic, nonionic, and amphoteric (such as zwitterionic) surfactants; cationic, anionic, nonionic, and amphoteric (such as zwitterionic) polymers other than polymers of the invention; polyols; proteins; provitamins; vitamins; dyes; tints; bleaches; pH adjusting agents, vegetable, animal, and synthetic waxes; and vegetable, animal, mineral, and synthetic oils. The compositions may also contain silicones, fatty esters, fatty alcohols, long chain hydrocarbons, emollients, lubricants, lanolin compounds, protein hydrolysates, and other protein derivatives.

In one embodiment, the at least one constituent is chosen from polymers, such as anionic, cationic, amphoteric (such as zwitterionic), and nonionic polymers and combinations thereof. As used herein, the term "polymer" refers to homopolymers and copolymers, the copolymers being derived from more than one type of monomer, such as from two, three, four, or more different monomer types.

The cationic polymers comprise cationic moieties or moieties that are convertible to cationic moieties. Suitable examples of cationic polymers, which can be used according to the present invention, are those that may be chosen from polymers comprising at least one group chosen from primary amine groups, secondary amine groups, tertiary amine groups, and quaternary amine groups, wherein the at least one group forms part of the polymer chain or is linked directly to it, having a weight average molecular weight ranging from about 500 to about 5,000,000, such as from about 1000 to about 3,000,000.

Among these polymers, mention may be made more particularly of the following cationic polymers:

(1) homopolymers and copolymers derived from monomers chosen from (meth)acrylic esters and (meth)acrylic amides comprising units of at least one of the following formulae: 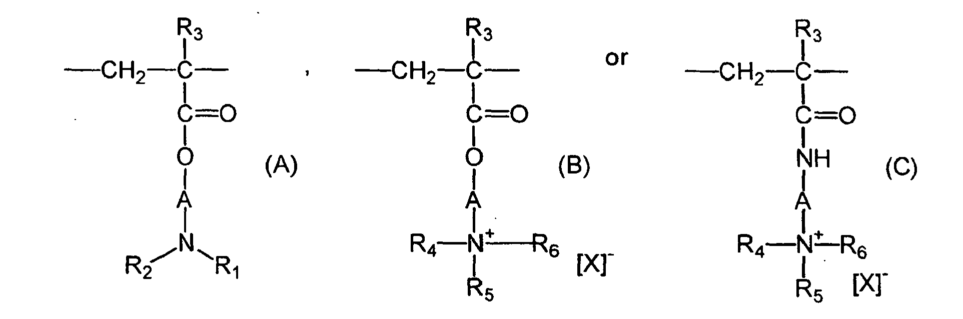 in which each R₃ is independently chosen from hydrogen and CH₃ groups; each A is independently chosen from linear and branched alkyl groups comprising 1 to 6 carbon atoms and hydroxyalkyl groups comprising 1 to 4 carbon atoms; each R₄, R₅, and R₆ is independently chosen from alkyl groups comprising 1 to 18 carbon atoms and benzyl groups; each R₁ and R₂ is independently chosen from hydrogen and alkyl groups comprising 1 to 6 carbon atoms; and each X⁻ is independently chosen from methyl sulfate anions and halide anions, such as chloride or bromide anions.

In one embodiment, the copolymers of family (1) further comprise at least one unit derived from monomers chosen from (meth)acrylamides, diacetone (meth)acrylamides, (meth)acrylamides substituted on the nitrogen by a group chosen from lower alkyls, (meth)acrylic acids, esters of (meth)acrylic acids, vinyllactams such as vinylpyrrolidone and vinyl-caprolactam, and vinyl esters.

Thus, mention may be made, among these cationic copolymers of the family (1), of:
- copolymers of acrylamide and of dimethylaminoethyl methacrylate quaternized with dimethyl sulfate or with a dimethyl halide, such as Hercofloc available from the company Hercules;
- copolymers of acrylamide and of methacryloyloxyethyltrimethylammonium chloride which are disclosed, for example, in EP-A-080,976 , and sold, for example, under the name Bina Quat P 100 by the company Ciba-Geigy;
- copolymers of acrylamide and of methacryloyloxyethyltrimethylammonium methosulfate, such as Reten available from the company Hercules;
- optionally quaternized vinylpyrrolidone/dialkyl-aminoalkyl (meth)acrylate copolymers, which are disclosed, for example, in French Patents 2,077,143 and 2,393,573, and sold, for example, under the name "Gafquat" by the company ISP, such as, for example, "Gafquat 734" or "Gafquat 755", or else the products named "Copolymer 845, 958 and 937";
- dimethylaminoethyl methacrylate/vinylcaprolactam/vinylpyrrolidone terpolymers, such as the product sold under the name Gaffix VC 713 by the company ISP, and
- the quaternized vinylpyrrolidone/dimethylamino-propylmethacrylamide copolymer, such as the product sold under the name "Gafquat HS 100" by the company ISP;

(2) the quaternized polysaccharides, disclosed more particularly in U.S. Patent Nos. 3,589,578 and 4,031,307, such as guar gums comprising cationic trialkylammonium cationic groups.

Such products are sold in particular under the trade names Jaguar C13 S, Jaguar C 15, and Jaguar C 17 by the company Meyhall.

(3) quaternized copolymers of vinylpyrrolidone and of vinylimidazole, such as the products sold by BASF under the name Luviquat TFC.

(4) chitosans or their salts. The salts, which can be used, are in particular chitosan acetate, lactate, glutamate, gluconate, or pyrrolidone-carboxylate.

Mention may be made, among these compounds, of the chitosan having a degree of deacetylation of 90.5% by weight sold under the name Kytan Crude Standard by the company Aber Technologies and the chitosan pyrrolidone-carboxylate sold under the name Kytamer PC by the company Amerchol.

(5) Cationic cellulose derivatives, such as the copolymers of cellulose and the cellulose derivatives grafted with a water-soluble quaternary ammonium monomer and disclosed in particular in U.S. Patent No. 4,131,576. Examples include hydroxyalkyl celluloses, for example hydroxymethyl, hydroxyethyl, and hydroxypropyl celluloses grafted in particular with a methacryloylethyltrimethylammonium, methacrylamidopropyltrimethylammonium, or diallyldimethylammonium salt.

The commercial products corresponding to this definition are more particularly the products sold under the name "Celquat L 200" and "Celquat H 100" by the company National Starch.

The anionic polymers, which can be used according to the present invention, are polymers comprising groups derived from carboxylic, sulfonic, and/or phosphoric acid and having a weight average molecular weight ranging from about 500 to about 5,000,000.

(1) The carboxyl groups may be contributed by unsaturated mono- and dicarboxylic acid monomers such as those corresponding to the formula: 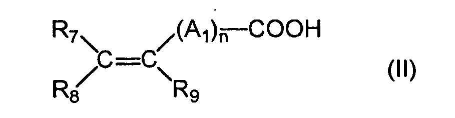 in which n is an integer ranging from 0 to 10; A₁ denotes a methylene group and when n is greater than 1, each A₁ is independently represented by -LCH₂-, where L is chosen form a valency bond and heteroatoms, such as oxygen and sulfur; R₇ is chosen from hydrogen, phenyl groups, and benzyl groups; R₈ is chosen from hydrogen, lower alkyl groups, and carboxyl groups; and R₉ is chosen from hydrogen, lower alkyl groups, -CH₂-COOH groups, phenyl groups, and benzyl groups.

As defined herein, a lower alkyl group denotes a group having 1 to 4 carbon atoms, such as methyl and ethyl.

The anionic polymers comprising carboxyl groups according to the invention may be chosen from:

A) Homopolymers and copolymers of (meth)acrylic acids or (meth)acrylic salts and in particular the products sold under the names Versicol E or K by the company Allied Colloid and Ultrahold by the company BASF, the copolymers of acrylic acid and of acrylamide sold in the form of their sodium salt under the names Reten 421, 423, or 425 by the company Hercules, and the sodium salts of polyhydroxycarboxylic acids.

B) Copolymers of (meth)acrylic acid with a monoethylenic monomer, such as ethylene, styrene, vinyl esters, and (meth)acrylic acid esters, optionally grafted onto a polyalkylene glycol, such as polyethylene glycol, and optionally crosslinked. Such polymers are disclosed in particular in French Patent 1,222,944 and German Application 2,330,956. The copolymers of this type comprising, in their chain, an optionally N-alkylated and/or hydroxyalkylated acrylamide unit, such as disclosed in particular in Luxembourg Patent Applications 75370 and 75371, or sold under the name Quadramer by the company American Cyanamid. Mention may also be made of copolymers of acrylic acid and of C₁-C₄ alkyl methacrylate and terpolymers of vinylpyrrolidone, of acrylic acid, and of C₁-C₂₀ alkyl methacrylate for example lauryl methacrylate, such as Acrylidone LM available from the company ISP, and methacrylic acid/ethyl acrylate/tert-butyl acrylate terpolymers, such as the product sold under the name Luvimer 100 P by the company BASF.

C) copolymers derived from crotonic acid, such as those comprising, in their chain, vinyl acetate or propionate units and optionally other monomers, such as (meth)allyl esters; vinyl ethers and vinyl esters of linear and branched saturated carboxylic acids comprising a long hydrocarbon chain, such as those comprising at least 5 carbon atoms, it optionally being possible for these polymers to be grafted and crosslinked; or alternatively vinyl and (meth)allyl esters of an α- or β-cyclic carboxylic acid. Such polymers are disclosed, inter alia, in French Patents 1,222,944, 1,580,545, 2,265,782, 2,265,781, 1,564,110, and 2.439.798. Commercial products coming within this class are the Resins 28-29-30, 26-13-14, and 28-13-10 sold by the company National Starch.

D) copolymers derived from C₄-C₈ monounsaturated carboxylic acids or anhydrides chosen from:
- copolymers comprising units derived from (i) at least one monomer chosen from maleic, fumaric, and itaconic acids and anhydrides thereof and (ii) at least one monomer chosen from vinyl esters, vinyl ethers, vinyl halides, phenylvinyl derivatives, acrylic acids, and acrylic acid esters, the anhydride functional groups of these copolymers optionally being monoesterified or monoamidated. Such polymers are disclosed in particular in U.S. Patent Nos. 2,047,398, 2,723,248, and 2,102,112 and GB 839,805 and in particular those sold under the names Gantrez AN or ES by the company ISP.
- copolymers comprising units derived from (i) at least one monomer chosen from maleic, citraconic, and itaconic anhydrides and (ii) at least one monomer chosen from (meth)allyl esters, optionally comprising in their chain at least one unit derived from groups chosen from (meth)acrylamide, α-olefin, (meth)acrylic ester, (meth)acrylic acid, and vinylpyrrolidone groups. The anhydride functional groups of these copolymers optionally are monoesterified or monoamidated.

These polymers are, for example, disclosed in French Patents 2,350,384 and 2,357,241.

E) polyacrylamides comprising carboxylate groups.

(2) The anionic polymers comprising sulfonic groups may be chosen from polymers comprising units, such as those derived from vinylsulfonic, styrenesulfonic, naphthalenesulfonic, and acrylamidoalkylsulfonic acids and their derivatives. These polymers may be chosen from:
- salts of polyvinylsulfonic acid having a weight average molecular weight that ranges from about 1000 to about 100,000, as well as the copolymers derived from at least one unsaturated comonomer, such as (meth)acrylic acids, their esters, acrylamides, their derivatives, vinyl ethers, and vinylpyrrolidone;
- salts of polystyrenesulfonic acid, the sodium salts having a weight average molecular weight ranging from about 100,000 to about 500,000, which are sold respectively under the names Flexan 500 and Flexan 130 by National Starch. These compounds are disclosed in Patent FR 2,198,719;
- salts of polyacrylamidesulfonic acids, including those mentioned in U.S. Patent No. 4,128,631, and more particularly the polyacrylamidoethylpropanesulfonic acid sold under the name Cosmedia Polymer HSP 1180 by Henkel.

In one embodiment, the anionic polymers are chosen from acrylic acid copolymers, such as the acrylic acid/ethyl acrylate/N-tert-butylacrylamide terpolymer sold under the name Ultrahold Strong by the company BASF; copolymers derived from crotonic acid, such as the vinyl acetate/vinyl tert-butylbenzoate/crotonic acid terpolymers and the crotonic acid/vinyl acetate/vinyl neododecanoate terpolymers sold under the name Resin 28-29-30 by the company National Starch; polymers derived from at least one monomer chosen from maleic, fumaric, and itaconic acids and anhydrides thereof and also from at least one monomer chosen from vinyl esters, vinyl ethers, vinyl halides, phenylvinyl derivatives, acrylic acid, and esters of acrylic acid, such as the monoesterified methyl vinyl ether/maleic anhydride copolymer sold under the name Gantrez ES 425 by the company ISP; copolymers of methacrylic acid and of methyl methacrylate sold under the name Eudragit L by the company Rohm Pharma; the copolymer of methacrylic acid and of ethyl acrylate sold under the name Luvimer MAEX or MAE by the company BASF; the vinyl acetate/crotonic acid copolymer sold under the name Luviset CA 66 by the company BASF; and the vinyl acetate/crotonic acid copolymer grafted by polyethylene glycol sold under the name Aristoflex A by the company BASF.

In another embodiment, the anionic polymers are chosen from the monoesterified methyl vinyl ether/maleic anhydride copolymer sold under the name Gantrez ES 425 by the company ISP; the acrylic acid/ethyl acrylate/N-tert-butylacrylamide terpolymer sold under the name Ultrahold Strong by the company BASF; the copolymers of methacrylic acid and of methyl methacrylate sold under the name Eudragit L by the company Rohm Pharma; the vinyl acetate/vinyl tert-butylbenzoate/crotonic acid terpolymers and the crotonic acid/vinyl acetate/vinyl neododecanoate terpolymers sold under the name Resin 28-29-30 by the company National Starch; the copolymer of methacrylic acid and of ethyl acrylate sold under the name Luvimer MAEX or MAE by the company BASF; and the vinyl-pyrrolidone/acrylic acid/lauryl methacrylate terpolymer sold under the name Acrylidone LM by the company ISP.

The amphoteric polymers, which can be used in accordance with the invention, may be chosen from polymers comprising X and Y units, distributed randomly in the polymer chain, where the X unit is chosen from units derived from at least one monomer comprising at least one basic function, in particular a basic nitrogen atom, and where the Y unit is chosen from units derived from at least one acidic monomer comprising at least one group chosen from carboxyl groups and sulfo groups, or else where each X and Y unit is independently chosen from groups derived from zwitterionic carboxybetaine and sulfobetaine monomers. In another embodiment, the amphoteric polymers, which can be used in accordance with the invention, may be chosen from polymers comprising X and Y units, each X and Y unit is independently chosen from at least one cationic polymer chain comprising at least one group chosen from primary amine groups, secondary amine groups, tertiary amine groups, and quaternary amine groups, in which at least one of the amine groups comprises a group chosen from carboxyl and sulfo groups linked by way of a hydrocarbon group, or else the X and Y units, which may be different or identical, form part of a chain of at least one polymer comprising an ∀,∃-dicarboxy ethylene unit, wherein at least one of the carboxyl groups has been reacted with a polyamine comprising at least one group chosen from primary and secondary amine groups.

In one embodiment, the amphoteric polymers corresponding to the definition given above are chosen from the following polymers:

(1) polymers resulting from the copolymerization of a monomer derived from a vinyl compound carrying a carboxyl group, such as (meth)acrylic acids, maleic acids, and α-chloracrylic acids, and of a basic monomer derived from a substituted vinyl compound comprising at least one basic atom, such as dialkylaminoalkyl (meth)acrylate and dialkylaminoalkyl (meth)acrylamide. Such compounds are disclosed in U.S. Patent No. 3,836,537.

(2) polymers comprising units derived from:
a) at least one monomer chosen from (meth)acrylamides substituted on the nitrogen with an alkyl group,
b) at least one acidic comonomer comprising at least one reactive carboxylic group, and
c) at least one basic comonomer, such as esters comprising at least one substituent chosen from primary, secondary, tertiary, and quaternary amine substituents of (meth)acrylic acids, and the product of quaternization of dimethylaminoethyl methacrylate with dimethyl or diethyl sulfate.

The at least one N-substituted (meth)acrylamide monomer recited in (a) is more particularly chosen from N-substituted (meth)acrylamides, wherein the alkyl groups comprise from 2 to 12 carbon atoms, such as N-ethylacrylamide, N-tert-butylacrylamide, N-tert-octylacrylamide, N-octylacrylamide, N-decylacrylamide, N-dodecylacrylamide, and the corresponding methacrylamides.

The at least one acidic comonomer recited in (b) is more particularly chosen from (meth)acrylic acids, crotonic acids, itaconic acids, maleic acids, fumaric acids, C₁-C₄ alkyl monoesters of maleic acid, C₁-C₄ alkyl monoesters of fumaric acid, C₁-C₄ alkyl monoesters of maleic anhydride, and C₁-C₄ alkyl monoesters of fumaric anhydride.

The at least one basic comonomer recited in (c) is more particualry chosen from aminoethyl, butylaminoethyl, N,N'-dimethylaminoethyl, and N-tert-butylaminoethyl methacrylates.

In one embodiment, the amphoteric polymer is chosen from the copolymers for which the CTFA name (4th Ed., 1991) is octylacrylamide/ acrylates/ butylaminoethyl methacrylate copolymer, such as the products sold under the name Amphomer or Lovocryl 47 by the company National Starch.

(3) crosslinked and alkylated polyamino amides partially or totally derived from polyamino amides of general formula: 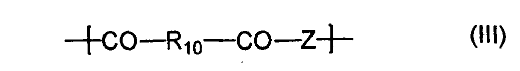 in which R₁₀ represents a divalent group derived either from a saturated dicarboxylic acid, from an aliphatic mono- or dicarboxylic acid comprising an ethylenic double bond, from an ester of a lower alkanol (having 1 to 6 carbon atoms) of these acids, or from a group derived from the addition of any one of the said acids with a bisprimary or bissecondary amine; and Z denotes a group of a bisprimary, mono- or bissecondary polyalkylenepolyamine and, for example, represents:

a) in the proportions of from about 60 mol% to 100 mol%, the group: 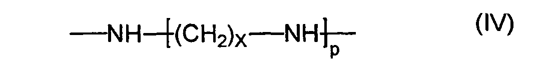 where x=2 and p=2 or 3, or else x=3 and p=2 and where this group derives from diethylenetriamine, triethylenetetraamine, or dipropylenetriamine;

b) in the proportions of from 0 mol% to about 40 mol%, the above group (IV), in which x=2 and p=1 and which derives from ethylenediamine, or the group derived from piperazine:

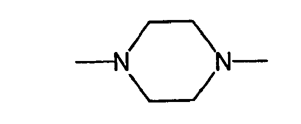

c) in the proportions of from 0 mol% to about 20 mol%, the -NH-(CH₂)₆-NH- group derived from hexamethylenediamine, these polyamino amines being crosslinked by addition of a difunctional crosslinking agent chosen from epihalohydrines, diepoxides, dianhydrides and bis-unsaturated derivatives, using from about 0.025 mol to about 0.35 mol of crosslinking agent per amine group of the polyamino amide and alkylated by the action of acrylic acid, chloroacetic acid or an alkane sultone, or salts thereof.

In one embodiment, the saturated carboxylic acids are chosen from acids having 6 to 10 carbon atoms, such as adipic acid, 2,2,4-trimethyladipic acid, 2,4,4-trimethyladipic acid, terephthalic acid, and acids comprising an ethylenic double bond such as, for example, acrylic acid, methacrylic acid and itaconic acid.

In one embodiment, the alkane sultones used in the alkylation are chosen from propane sultone and butane sultone and the salts of the alkylating agents are chosen from sodium and potassium salts.

(4) polymers comprising zwitterionic units of formula: 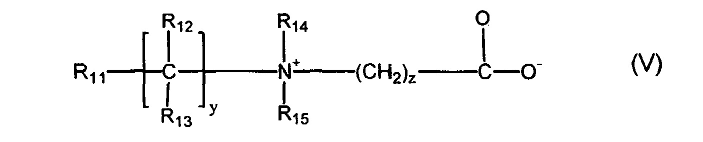 in which R₁₁ is chosen from polymerizable unsaturated groups such as an (meth)acrylate and (meth)acrylamide groups; y and z are independently chosen from integers ranging from 1 to 3; R₁₂ and R₁₃ are independently chosen from hydrogen, methyl groups, ethyl groups, and propyl groups; R₁₄ and R₁₅ are independently chosen from hydrogen and alkyl groups, wherein the sum of the carbon atoms in R₁₄ and R₁₅ is less than or equal to 10.

The polymers comprising such units may further comprise units derived from non-zwitterionic monomers, such as dimethylaminoethyl (meth)acrylate, diethylaminoethyl (meth)acrylate, alkyl (meth)acrylates, (meth)acrylamides, and vinyl acetates.

Mention may be made, by way of example, of the methyl methacrylate/ methyl dimethylcarboxymethylammonioethyl methacrylate copolymer, such as the product sold under the name Diaformer Z301 by the company Sandoz.

(5) polymers derived from chitosan comprising monomer units corresponding to the following formulae: 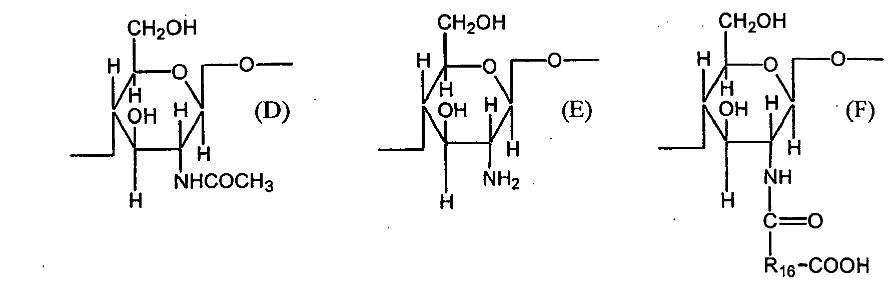 the unit D being present in proportions ranging from 0% to about 30%, the unit E in proportions ranging from about 5% to about 50% and the unit F in proportions ranging from about 30% to about 90%, it being understood that, in this unit F, R₁₆ represents a group of formula: 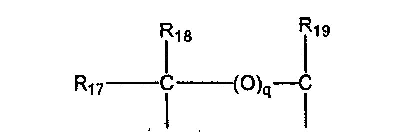 in which, if q=0, R₁₇, R₁₈, and R₁₉, which are identical or different, are chosen from hydrogen, methyl groups, hydroxyl groups, acetoxy groups, amino residues, monoalkylamine residues, and dialkylamine residues, optionally interrupted by one or more nitrogen atoms and/or optionally substituted by one or more amine, hydroxyl, carboxy, alkylthio, or sulfo groups, and alkylthio residues in which the alkyl group carries an amino residue, at least one of R₁₇, R₁₈, and R₁₉ being, in this case, hydrogen; or, if q=1, R₁₇, R₁₈, and R₁₉ each represent hydrogen, and the salts formed by these compounds with bases or acids.

(6) Polymers derived from the N-carboxyalkylation of chitosan, such as the N-(carboxymethyl)chitosan or the N-(carboxybutyl)chitosan sold under the name "Evalsan" by the company Jan Dekker.

(7) Polymers corresponding to the general formula (VI), for example disclosed in French Patent 1,400,366: 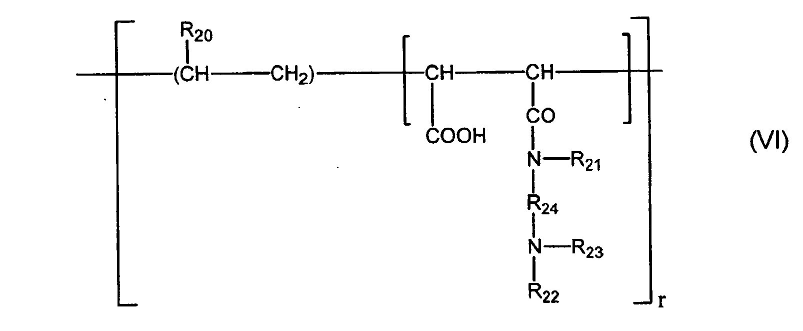 in which R₂₀ is chosen from hydrogen, CH₃O, CH₃CH₂O, and phenyl groups; R₂₁ is chosen from hydrogen and lower alkyl groups such as methyl or ethyl; R₂₂ is chosen from hydrogen and lower alkyl groups such as methyl or ethyl; and R₂₃ is chosen from lower alkyl groups such as methyl or ethyl and groups corresponding to the formula: -R₂₄-N(R₂₂)₂, where R₂₄ is chosen from -CH₂-CH₂-, -CH₂-CH₂-CH₂-, and -CH₂-CH(CH₃)- groups and R₂₂ is the same as above, and the higher homologues of these groups comprising up to 6 carbon atoms.

(8) Amphoteric polymers of the -D-X-D-X- type chosen from:

a) polymers obtained by reaction of chloracetic acid or sodium chloracetate with compounds comprising at least one unit of formula:

-D-X-D-X-D- (VII)

where D denotes a group 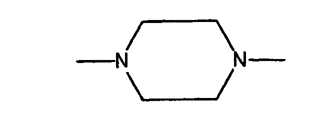 and X denotes the symbol E or E'. E and E', which are identical or different, denote a divalent group chosen from straight- and branched-chain alkylene groups comprising up to 7 carbon atoms in the main chain, which is unsubstituted or substituted by hydroxyl groups and which can additionally comprise oxygen, nitrogen, or sulfur atoms or 1 to 3 aromatic and/or heterocyclic rings; the oxygen, nitrogen, and sulfur atoms being present in the form of ether, thioether, sulfoxide, sulfone, sulfonium, alkylamine or alkenylamine groups, benzylamine, amine oxide, quaternary ammonium, amide, imide, alcohol, ester, and/or urethane groups.

b) Polymers of formula:

-D-X-D-X- (VII')

in which D denotes a group 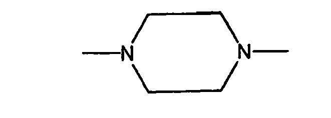 and X denotes the symbol E or E', where X denotes E' at least once, E has the meaning indicated above, and E' is a divalent group chosen from straight- and branched-chain alkylene groups having up to 7 carbon atoms in the main chain, which is substituted or unsubstituted by one or more hydroxyl groups and which comprises one or more nitrogen atoms, the nitrogen atom being substituted by an alkyl chain optionally interrupted by an oxygen atom and necessarily comprising one or more carboxyl functional groups or one or more hydroxyl functional groups and
wherein the polymer of formula VII' is betainized by reaction with chloracetic acid or sodium chloracetate.

(9) (C₁-C₅)alkyl vinyl ether/maleic anhydride copolymers, which are partially modified by semiamidation with an N,N-dialkylaminoalkylamine, such as N,N-dimethylaminopropylamine, or by semiesterification with an N,N-dialkanolamine. These copolymers can also comprise other vinyl comonomers, such as vinylcaprolactam.

In one embodiment, the amphoteric polymers according to the invention are chosen from family (3), such as the copolymers with the CTFA name (4^{th} Ed. 1991) of octylacrylamide/acrylates/butylaminoethyl methacrylate copolymer, such as the products sold under the names Amphomer, Amphomer LV 71, or Lovocryl 47 by the company National Starch, and family (4), such as the copolymer of methyl methacrylate/dimethyl carboxymethylammonio methyl ethylmethacrylate, sold, for example, under the name Diaformer Z301 by the company Sandoz.

The nonionic polymers, which can be used according to the present invention, are chosen, for example, from:
- vinylpyrrolidone homopolymers;
- copolymers of vinylpyrrolidone and of vinyl acetate;
- polyalkyloxazolines, such as the polyethyloxazolines sold by the company Dow Chemical under the names PEOX 50,000, PEOX 200,000, and PEOX 500,000;
- vinyl acetate homopolymers, such as the product sold under the name Appretan EM by the company Hoechst or the product sold under the name Rhodopas A 012 by the company Rhône-Poulenc;
- copolymers of vinyl acetate and of acrylic ester, such as the product sold under the name Rhodopas AD 310 by Rhône-Poulenc;
- copolymers of vinyl acetate and of ethylene, such as the product sold under the name Appretan TV by the company Hoechst;
- copolymers of vinyl acetate and of maleic ester, for example of dibutyl maleate, such as the product sold under the name Appretan MB Extra by the company Hoechst;
- copolymers of polyethylene and of maleic anhydride;
- alkyl acrylate homopolymers and alkyl methacrylate homopolymers, such as the product sold under the name Micropearl RQ 750 by the company Matsumoto or the product sold under the name Luhydran A 848 S by the company BASF;
- acrylic ester copolymers such as, for example, copolymers of alkyl (meth)acrylates, such as the products sold by the company Rohm & Haas under the names Primal AC-261 K and Eudragit NE 30 D, by the company BASF under the names Acronal 601, Luhydran LR 8833 or 8845, and by the company Hoechst under the names Appretan N 9213 or N 9212;
- copolymers of acrylonitrile and of a nonionic monomer chosen, for example, from butadiene and alkyl (meth)acrylates; mention may be made of the products sold under the names Nipol LX 531 B by the company Nippon Zeon or those sold under the name CJ 0601 B by the company Rohm & Haas;
- polyurethanes, such as the products sold under the names Acrysol RM 1020 or Acrysol RM 2020 by the company Rohm & Haas, and the products Uraflex XP 401 UZ and Uraflex XP 402 UZ by the company DSM Resins;
- copolymers of alkyl acrylate and of urethane, such as the product 8538-33 by the company National Starch;
- polyamides, such as the product Estapor LO 11 sold by the company Rhône-Poulenc.
- nonionic guar gums that are chemically modified or unmodified.

The unmodified nonionic guar gums are, for example, the products sold under the name Vidogum GH 175 by the company Unipectine and under the name Jaguar C by the company Meyhall.

The modified nonionic guar gums, which may be used according to the invention, are, for example, modified with C₁-C₆ hydroxyalkyl groups. Examples, which may be mentioned, are hydroxymethyl, hydroxyethyl, hydroxypropyl, and hydroxybutyl groups.

These guar gums are well known in the prior art and may be prepared, for example, by reacting corresponding alkene oxides such as, for example, propylene oxides with guar gum so as to obtain a guar gum modified with hydroxypropyl groups.

Such nonionic guar gums, optionally modified with hydroxyalkyl groups, are sold, for example, under the trade names Jaguar HP8, Jaguar HP60, Jaguar HP120, Jaguar DC 293, and Jaguar HP 105 by the company Meyhall and under the name Galactosol 4H4FD2 by the company Aqualon.

The alkyl groups in the nonionic polymers comprise from 1 to 6 carbon atoms, except where otherwise mentioned.

According to the invention, it is also possible to use polymers of grafted silicone type comprising a polysiloxane portion and a portion comprising a non-silicone organic chain, one of the two portions constituting the main chain of the polymer and the other being grafted onto the main chain. These polymers are disclosed, for example, in EP-A-0,412,704, EP-A-0,412,707, EP-A-0,640,105, WO 95/00578, EP-A-0,582,152, and WO 93/23009 and U.S. Patent Nos. 4,693,935, 4,728,571, and 4,972,037. These polymers are, for example, anionic or nonionic.

Such polymers are, for example, copolymers which can be obtained by radical polymerization from the monomer mixture comprising:
a) about 50% to about 90% by weight of tert-butyl acrylate;
b) 0% to about 40% by weight of acrylic acid;
c) about 5% to about 40% by weight of silicone macromer of formula: 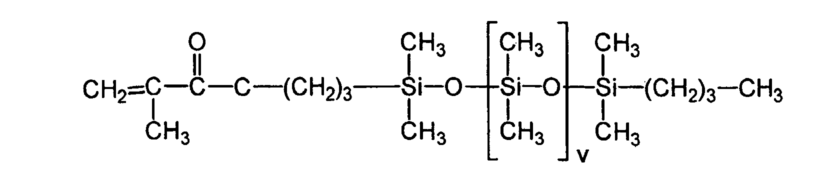
where v is a number ranging from 5 to 700; the percentages by weight being calculated with respect to the total weight of the monomers.

Other examples of grafted silicone polymers are, in particular, polydimethylsiloxanes (PDMSs) onto which are grafted, via a thiopropylene-type connecting chain, mixed polymer units of the poly(meth)acrylic acid type and of the poly(alkyl (meth)acrylate) type and polydimethylsiloxanes (PDMSs) onto which are grafted, via a thiopropylene-type connecting chain, polymer units of the poly(isobutyl (meth)acrylate) type.

It is also possible to use, as polymers, functionalized or non-functionalized and silicone-comprising or non-silicone-comprising polyurethanes.

Examples of useful polyurethanes include those disclosed in Patents EP 0,751,162, EP 0,637,600, FR 2,743,297, EP 0,648,485, EP 0,656,021, WO 94/03510, and EP 0,619,111.

In a further embodiment, the polymers may be used in solubilized form or may be in the form of dispersions of solid or liquid particles (latex or pseudo-latex).

The composition according to the invention may be vaporizable, for example by a pump, or may be a pressurized aerosol composition. It may be vaporizable by a dispensing valve controlled by a dispensing head, which in turn comprises a nozzle, which vaporizes the aerosol composition. A vaporizable composition according to the invention comprises an appropriate solvent. Advantageously, the appropriate solvent comprises at least one solvent chosen from water and lower alcohols. In accordance with the invention, the term lower alcohol means a C₁ to C₄ aliphatic alcohol, such as ethanol.

When the vaporizable composition according to the invention is an aerosol composition, it additionally comprises an appropriate amount of propellant. The propellant comprises compressed or liquefied gases, which are normally employed for the preparation of aerosol compositions. Suitable gasses include compressed air, carbon dioxide, nitrogen, and gases, which may be soluble in the composition, such as dimethyl ether, fluorinated or non-fluorinated hydrocarbons, and mixtures thereof.

The present invention additionally provides an aerosol device comprising a vessel comprising an aerosol composition, which comprises a liquid phase (or juice) comprising at least one reshapable hair styling material, as described above, in an appropriate medium and a propellant, and a dispenser, such as a dispensing valve, for dispensing said aerosol composition from the vessel.

The present invention additionally provides a method of treating keratinous fibers, especially hair, in which the reshapable hair styling composition according to the invention, as described above, is applied to the hair before, during, or after the shaping of the hairstyle.

The present invention additionally provides the use of a composition as described above in, or for the preparation of, a cosmetic reshapable hair styling formulation.

The determination of whether a composition with an (meth)acrylic copolymer according to the invention can provide a reshapable effect can be determined by an *in vivo* test.

Where the composition is in the form of a lotion, for example, the *in vivo* test proceeds as follows. The hair of the model is washed and then divided into two symmetrical portions, the right and the left sides. The composition is applied to one side of the head of the model, while a reference composition is applied to the other side of the head. The reference composition may, for example, be chosen from water, an existing commercial product, or another composition under study. The hairdresser dries and styles both sides of the head. The two sides of the head are separately evaluated for the styling effect, the cosmetic properties, and the reshapable effect. For example, once dried, the hair is brushed in different directions to remove the original styling. The hair is then brushed to restore the original styling. The process of removing the styling, restoring the styling, and evaluating the success of restoring the styling is repeated at least one more time to determine whether the composition is a reshapable hair styling composition. A reshapable hair styling composition permits (1) the original hair styling to be restored after brushing and (2) the creation of a new hair styling after brushing, which may also be restored after brushing. If the composition to be evaluated is in another form, the *in vivo* test can be appropriately modified by one skilled in the art.

It is understood that the person skilled in the art would recognize that not all formulations would provide reshapable effect for all hair types during *in vivo* testing and will know how to formulate and evaluate reshapable hair styling compositions in view of the various hair parameters, such as length (short versus long), diameter (thin versus thick), structure (curly versus straight), condition (oily, dry, or normal); and whether the hair is colored, bleached, permed, or straightened. Thus, *in vivo* testing may require testing on 10-20 different individuals.

Other than in the operating examples, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Any numerical value inherently contains certain errors necessarily resulting from the standard deviation found in their respective testing measurements.

EXAMPLES:

Hair compositions according to the invention were produced with different (meth)acrylic emulsions.

### 1) Preparation of the acrylic emulsions:

### Example 1:

A mixture of 300 grams 2-ethyl hexyl acrylate (2-EHA), 175 grams n-butyl acrylate (BA), and 25 grams 2-hydroxy ethyl methacrylate (HEMA) was prepared yielding 500 grams of a monomer solution containing 60/35/5 parts 2-EHA/BA/HEMA. Of the total monomer solution, 50 grams was charged into a two liter split resin flask along with 380 grams of deionized water and 0.5 gram of RHODACAL DS-10 (sodium dodecyl benzene sulfonate surfactant commercially available from Rhône-Poulenc). The head was placed on the flask and a thermocouple, nitrogen inlet, and mechanical stirrer attached. The contents were heated with infra red lamps to about 60 °C while stirring at 350 rpm. A solution of 1 gram potassium persulfate initiator in 20 grams deionized water was charged, the flask sealed, and a vacuum pulled on the flask four times, breaking it each time with nitrogen. The flask was held at 60 °C for 20 minutes, then heated to 80 °C over 10 minutes to yield a seed polymer. A pre-emulsion of the remaining 450 grams of the monomer solution was prepared by charging a solution of 4.5 grams of sodium dodecyl benzene sulfonate in 211 grams of deionized water to it and stirring under nitrogen. This pre-emulsion was added dropwise to the two liter split resin flask containing the seed polymer at a rate of 6 grams per minute. The addition took almost 2 hours. After the addition, the stirring rate was reduced to 200 rpm and the reaction held at 80 °C for two hours, then the resulting latex was filtered through doubled over cheesecloth into a jar. Low levels of coagulum were noted around the thermocouple and stirring paddle.

### Prophetic Example 2:

A mixture of 300 grams (2-EHA), 100 grams iso-butyl acrylate (IBA), 75 grams (BA), and 25 grams (HEMA) is prepared yielding 500 grams of a monomer solution containing 60/20/15/5 parts 2-EHA/IBA/BA/HEMA. Of the total monomer solution, 50 grams is charged into a two liter split resin flask along with 380 grams of deionized water and 0.5 gram of RHODACAL DS-10 (sodium dodecyl benzene sulfonate surfactant commercially available from Rhône-Poulenc). The head is placed on the flask and a thermocouple, nitrogen inlet, and mechanical stirrer are attached. The contents are heated with infra red lamps to about 60 °C while stirring at 350 rpm. A solution of 1 gram potassium persulfate initiator in 20 grams deionized water is charged, the flask is sealed, and a vacuum is pulled on the flask four times, breaking it each time with nitrogen. The flask is held at 60 °C for 20 minutes, then is heated to 80 °C over 10 minutes to yield a seed polymer. A pre-emulsion of the remaining 450 grams of the monomer solution is prepared by charging a solution of 4.5 grams of sodium dodecyl benzene sulfonate in 211 grams of deionized water to it and stirring under nitrogen. This pre-emulsion is added dropwise to the two liter split resin flask containing the seed polymer at a rate of 6 grams per minute. The addition takes almost 2 hours. After the addition, the stirring rate is reduced to 200 rpm and the reaction is to be held at 80 °C for two hours, then the resulting latex will be filtered through doubled over cheesecloth into a jar.

### Prophetic Examples 3:

A 50/50 mixture of the emulsion from Example 1 and a dispersion comprising AQ 1350 by the Eastman Chemical Co. as disclosed in WO 98/38969 can be made.

### 2) Preparation of a hair styling composition:

A hair styling composition in accordance with the invention was prepared using the components and amounts in weight percent listed hereafter.

| Formulation A: | |
|---|---|
| Example 1 | 4 g active material |
| Water | qsp 100g |

Formulation A was applied to the hair in a non-rinse process. After drying, a visible reshapable effect was observed.

## Claims

1. A reshapable hair styling composition comprising at least one (meth)acrylic copolymer, wherein said at least one (meth)acrylic copolymer comprises:
(a) units derived from at least one monomer chosen from butyl (meth)acrylate monomers,
(b) units derived from at east one monomer chosen from hydroxy alkyl (meth)acrylate monomers, and
(c) units derived from at least one co-polymerizable monomer other than said (a) and (b) monomers,
wherein said composition provides a reshapable effect and is a non-rinse composition, and
(a) is n-butyl acrylate, and
(b) 2-hydroxy ethyl methacrylate, and
(c) is 2-ethyl hexylacrylate.

2. The composition according to claim 1, wherein the composition further comprises a cosmetically acceptable vehicle.

3. The composition according to claim 1, wherein said at least one (meth)acrylic copolymer is an emulsic. n.

4. The composition according to claim 1, wherein said at least one (meth)acrylic copolymer is crosslinked with at least one polyfunctional cross-linking agent.

5. The composition according to claim 4, wherein said at least one polyfunctional crosslinking agent s chosen from divinylbenzene, alkyl diacrylates, alkyl triacrylates, alkyl tetracrylates, and monoethylenically unsaturated aromatic ketones.

6. The composition according to claim 5, wherein said at least one polyfunctional crosslinking agent is chosen from multifunctional aziridine amides and metal ion crosslinkers.

7. The composition according to claim 3, wherein said emulsion has a pH less than or equal to 7.

8. The composition according to claim 1, wherein said at least one (meth)acrylic copolymer is chosen from nonionic and weakly anionic (meth)acrylic copolymers.

9. The composition according to claim 1, wherein said at least one (meth)acrylic copolymer is present it an amount ranging from about 0.01 to about 40 weight percent of the total weight pe cent of the composition.

10. The composition according to claim 9, wherein the amount of said at least one (meth)acrylic copolymer ranges from about 0.1 to about 15 weight percent.

11. The composition according to claim 1, wherein said at least one (meth)acrylic copolymer has a Tg ranging from about -100 °C to about 15°C.

12. The composition according to claim 1, further comprising at least one constituent chosen from reducing agents; silanes; fatty substances; thickeners; plasticizers; anti-foaming agents; hydrating agents; fillers; sunscreens; active haircare agents; perfumes; preservatives; cationic, anionic, nonionic, and amphoteric surfactants; cationic, anionic, nonionic, and amphoteric polymers; polyols; proteins; provitamins; vitamins; dyes; tints; bleaches; pH adjusting agents; silicones; esters; vegetable, anima, and synthetic waxes; and vegetable, animal, mineral, and synthetic oils.

13. The composition according to claim 12, wherein said at least one constituent is chosen from cationic, anionic, nonionic, and amphoteric polymers.

14. A reshapable hair styling composition according to claim 1,
wherein said composition provides a reshapable effect and is a non-rinse composition in a form chosen from prays, aerosols, mousses, gels, sticks, muds, lotions, creams, dispersions, and emulsions.

15. An aerosol device comprising a vessel, which comprises an aerosol composition according to any of the preceding claims.

16. A method of cosmetically treating hair, comprising applying to the hair before, during, or after shaping of a hairstyle of said hair a composition according to any of claims 1 to 14.

## Patentansprüche

1. Hairstyling-Zusammensetzung, die erneut modelliert werden kann und die mindestens ein (Meth)acrylcopolymer enthält, wobei dieses zumindest eine (Meth)acrylcopolymer enthält:
(a) Einheiten, die von mindestens einem Monomer abgeleitet sind, das unter Butyl(meth)acrylatmonomeren ausgewählt ist,
(b) Einheiten, die von zumindest einem Monomer abgeleitet sind, das unter Hydroxyalkyl(meth)acrylatmonomeren ausgewählt ist, und
(c) Einheiten, die von zumindest einem copolymerisierbaren Monomer stammen, das von den Monomeren (a) und (b) verschieden ist,
wobei diese Zusammensetzung erneut modellierbar ist und es sich um eine Zusammensetzung handelt, die nicht ausgespült wird, und wobei es sich bei
(a) um n-Butylacrylat, und
(b) 2-Hydroxyethylmethacrylat und
(c) 2-Ethylhexylacrylat handelt.

2. Zusammensetzung nach Anspruch 1, wobei die Zusammensetzung ferner mindestens einen kosmetisch akzeptablen Träger enthält.

3. Zusammensetzung nach Anspruch 1, wobei das zumindest eine (Meth)acrylcopolymer in einer Emulsion enthalten ist.

4. Zusammensetzung nach Anspruch 1, wobei das zumindest eine (Meth)acrylcopolymer mit mindestens einem polyfunktionellen Vernetzungsmittel vernetzt ist.

5. Zusammensetzung nach Anspruch 4, wobei das zumindest eine polyfunktionelle Vernetzungsmittel unter Divinylbenzol, Alkyldiacrylaten, Alkyltriacrylaten, Alkyltetraacrylaten und monoethylenisch ungesättigten aromatischen Ketonen ausgewählt ist.

6. Zusammensetzung nach Anspruch 5, wobei das zumindest eine polyfunktionelle Vernetzungsmittel unter multifunktionellen Aziridinamiden und Metallionen-Crosslinkern ausgewählt ist.

7. Zusammensetzung nach Anspruch 3, wobei die Emulsion einen pH-Wert von 7 oder darunter aufweist.

8. Zusammensetzung nach Anspruch 1, wobei das zumindest eine (Meth)acrylcopolymer unter nichtionischen und schwach anionischen (Meth)acrylcopolymeren ausgewählt ist.

9. Zusammensetzung nach Anspruch 1, wobei das zumindest eine (Meth)acrylcopolymer in einer Menge von etwa 0,01 bis etwa 40 Gew.-% des Gesamtgewichts der Zusammensetzung enthalten ist.

10. Zusammensetzung nach Anspruch 9, wobei der Mengenanteil des zumindest einen (Meth)acrylcopolymers im Bereich von etwa 0,1 bis 15 Gew.-% liegt.

11. Zusammensetzung nach Anspruch 1, wobei das zumindest eine (Meth)acrylcopolymer eine Tg von etwa -100 bis etwa 15 °C auf weist.

12. Zusammensetzung nach Anspruch 1, die ferner zumindest einen Bestandteil enthält, der unter Reduktionsmitteln; Silanen; Fettsubstanzen; Verdickungsmitteln; Weichmachern; Schaumverhütungsmitteln; Hydratisierungsmitteln; Füllstoffen; Sonnenschutzfiltern; Konditioniermitteln für die Haare; Parfums; Konservierungsmitteln; kationischen, anionischen, nichtionischen und amphoteren grenzflächenaktiven Stoffen; kationischen, anionischen, nichtionischen und amphoteren Polymeren; Polyolen; Proteinen; Provitaminen; Vitaminen; Farbstoffen; Farbmitteln; Bleichmitteln; Stoffen zur Einstellung des pH-Werts; Siliconen; Estern; pflanzlichen, tierischen und synthetischen Wachsen; und pflanzlichen, tierischen, mineralischen und synthetischen Ölen ausgewählt ist.

13. Zusammensetzung nach Anspruch 12, wobei der zumindest eine Bestandteil unter den kationischen, anionischen, nichtionischen und amphoteren Polymeren ausgewählt ist.

14. Hairstyling-Zusammensetzung, die erneut modelliert werden kann, nach Anspruch 1, wobei diese Zusammensetzung neu formbar ist und es sich um eine Zusammensetzung handelt, die nicht ausgespült wird, in einer Form, die ausgewählt ist unter: Sprays, Aerosolen, Schäumen, Gelen, Sticks, Packungen, Lotionen, Cremes, Dispersionen und Emulsionen.

15. Aerosol mit einem Behälter, der eine Aerosolzusammensetzung nach einem der vorhergehenden Ansprüche enthält.

16. Verfahren zur kosmetischen Behandlung der Haare, das das Auftragen einer Zusammensetzung nach einem der Ansprüche 1 bis 14 auf die Haare umfasst, bevor, während oder nachdem die Frisur modelliert wurde.

## Revendications

1. Composition de coiffage déformable comprenant au moins un copolymère (méth)acrylique, **caractérisée en ce que** ledit au moins un copolymère (méth)acrylique comprend:
(a) des motifs issus d'au moins un monomère choisi parmi les monomères de (méth)acrylate de butyle,
(b) des motifs issus d'au moins un monomère choisi parmi les monomères de (méth)acrylate d'hydroxyalkyle, et
(c) des motifs issus d'au moins un monomère co-polymérisable autre que lesdits monomères (a) et (b),
**en ce que** ladite composition a un effet déformable et est une composition non-rincée, et
(a) est l'acrylate de n-butyle,
(b) est le méthacrylate de 2-hydroxyéthyle, et
(c) est l'hexylacrylate de 2-éthyle.

2. Composition selon la revendication 1, **caractérisée en ce que** la composition comprend en outre un véhicule cosmétiquement acceptable.

3. Composition selon la revendication 1, **caractérisée en ce que** ledit au moins un copolymère (méth)acrylique est une émulsion.

4. Composition selon la revendication 1, **caractérisée en ce que** ledit au moins un copolymère (méth)acrylique est réticulé avec au moins un agent de réticulation polyfonctionnel.

5. Composition selon la revendication 4, **caractérisée en ce que** ledit au moins un agent de réticulation polyfonctionnel est choisi parmi le divinyl-benzène, les diacrylates d'alkyle, les triacrylates d'alkyle, les tétraacrylates d'alkyle et les cétones aromatiques à insaturation mono-éthylénique.

6. Composition selon la revendication 5, **caractérisée en ce que** ledit au moins un agent de réticulation polyfonctionnel est choisi parmi les aziridine-amides multifonctionnels et les agents de réticulation ions métalliques.

7. Composition selon la revendication 3, **caractérisée en ce que** ladite émulsion a un pH inférieur ou égal à 7.

8. Composition selon la revendication 1, **caractérisée en ce que** ledit au moins un copolymère (méth)acrylique est choisi parmi les copolymères (méth)acryliques non-ioniques et faiblement anioniques.

9. Composition selon la revendication 1, **caractérisée en ce que** ledit au moins un copolymère (méth)acrylique est présent dans une quantité allant d'environ 0,01 à environ 40 % en poids du poids total en pourcentage de la composition.

10. Composition selon la revendication 9, **caractérisée en ce que** la quantité dudit au moins un copolymère (méth)acrylique va d'environ 0,1 à environ 15 % en poids.

11. Composition selon la revendication 1, **caractérisée en ce que** ledit au moins un copolymère (méth)acrylique a une Tg allant d'environ -100°C à environ 15°C.

12. Composition selon la revendication 1 comprenant, en outre, au moins un constituant choisi parmi les agents réducteurs ; les silanes ; les matières grasses ; les épaississants ; les plastifiants ; les agents anti-mousse ; les agents hydratants ; les charges; les filtres solaires; les agents actifs de soins des cheveux; les parfums ; les conservateurs; les tensioactifs cationiques, anioniques, non-ioniques et amphotères; les polymères cationiques, anioniques, non-ioniques et amphotères ; les polyols ; les protéines; les provitamines ; les vitamines; les colorants; les produits de teinture ; les produits de décoloration ; les agents de modification du pH ; les silicones ; les esters ; les cires végétales, animales et synthétiques ; et les huiles végétales, animales, minérales et de synthèse.

13. Composition selon la revendication 12, **caractérisée en ce que** ledit au moins un constituant est choisi parmi les polymères cationiques, anioniques, non-ioniques et amphotères.

14. Composition de coiffage déformable selon la revendication 1, **caractérisée en ce que** ladite composition a un effet déformable et est une composition non-rincée sous une forme choisie parmi les sprays, les aérosols, les mousses, les gels, les bâtons, les boues, les lotions, les crèmes, les dispersions et les émulsions.

15. Dispositif aérosol comprenant un récipient, qui comprend une composition aérosol selon l'une quelconque des revendications précédentes.

16. Procédé de traitement cosmétique des cheveux, comprenant l'application sur les cheveux avant, pendant ou après la mise en forme d'une coiffure desdits cheveux d'une composition selon l'une quelconque des revendications 1 à 14.
